# EUROPEAN PATENT SPECIFICATION

(11) **EP 1 310 565 B1**
(45) Date of publication and mention of the grant of the patent: **09.01.2008**
(21) Application number: 01126786.1
(22) Date of filing: 09.11.2001
(51) Int. Cl.: C12Q 1/68, C12Q 1/70

(54) **A process for detecting nucleic acid target molecules using an enzyme-linked probe capture assay and a kit therefor**
Verfahren zur Detektion von Nukleinsäure-Zielmolekülen bei dem Enzym verbundenen Sonde Einfangtest sowie geeignetes Testsystem
Procédé pour détecter des molécules d'acides nucléiques cibles utilisant un test de capture de sonde liée à une enzyme et kit pour sa mise en oeuvre

(43) Date of publication of application: 14.05.2003
(73) Proprietor: Hong Kong DNA Chips Limited, Kowloon, Hong Kong Special Administrative Region (CN)
(72) Inventor: Yu, Albert Cheung-Hoi, Kowloon, Hong Kong Special Admin. Region (CN); Lau, Lok-Ting, Kowloon, Hong Kong Special Admin. Region (CN); Collins, Richard Anthony, Kowloon, Hong Kong Special Admin. Region (CN); Chan, Ka-Yan, Kowloon, Hong Kong Special Admin. Region (CN); Ko, Lung-Sang, Kowloon, Hong Kong Special Admin. Region (CN); Lin, Sau-Wah, Kowloon, Hong Kong Special Admin. Region (CN)
(74) Representative: Epping - Hermann - Fischer

(56) References cited:
- EP-A- 0 628 568
- WO-A-02/29118
- WO-A-93/15221
- US-A- 5 639 609
- SUAREZ DL ET AL: "Comparisons of highly virulent H5N influenza A viruses isolated from humans and chickens from Hong Kong." JOURNAL OF VIROLOGY, vol. 72, no. 8, August 1998 (1998-08), pages 6678-6688, XP002201888 & DATABASE GENBANK [Online] NCBI; SUAREZ DL ET AL: "Influenza A virus A/Chicken/Hong Kong/220/97 hemagglutinin subtype H5 (H5) gene, complete cds." Database accession no. AF046080
- SAMBROOK J AND RUSSELL DW: "Molecular cloning: a laboratory manual" January 2001 (2001-01) , COLD SPRING HARBOR LABORATORY PRESS , COLD SPRING HARBOR, NEW YORK XP002201867 * see Vol. 3, pages A9.38 to A9.40 *
- AMANNA RAHMAN ET AL.: "Development and evaluation of NASBA and "real-time" RT-PCR for diagnosis of viral respiratory tract infection" JOURNAL OF CLINICAL VIROLOGY, vol. 18, 2000, page 174,
- SHENGQIANG L. ET AL: "Recombinant influenza A virus vaccines for the pathogenic human A/Hong Kong/97 (H5N1) viruses" THE JOURNAL OF INFECTIOUS DISEASES, vol. 179, 1999, pages 1132-1138,

## Description

### Field of the Invention

This invention relates to a process for detecting nucleic acid target molecules, especially amplified nucleic acid products following nucleic acid sequence-based amplification (NASBA) using an enzyme-linked probe capture assay according to claim 1, and a kit therefore according to claim 15.

### Background of the Invention

Many techniques have been described for detecting nucleic acids. These include techniques based on the absorption characteristics of the nucleic acid molecule (e.g. ratio of absorbance at 260 nm and 280 nm), the ability of the nucleic acid to interact with various dyes and intercalating agents (e.g. ethidium bromide, propidium iodide) and the ability of the nucleic acid to bind to various surfaces (e.g. nylon). The ability to amplify specific regions of nucleic acid many hundreds of times is the key to successful detection. Several methods have been described allowing amplification of nucleic acid target molecules (e.g. polymerase chain reaction (PCR), reverse transcriptase-PCR (RT-PCR), branched DNA signal amplification assay, and NASBA among others).

It is the property of denatured single stranded DNA or RNA to hybridise to a complementary oligonucleotide probe that has been of particular use in molecular biology. This property has been exploited in techniques such as NASBA. NASBA is an enzyme-based method for the amplification of nucleic acid (Romano et al., 1996. Clin. Lab. Med. 16 (1), 89-103). The technique is particularly suited for the amplification of single stranded RNA and has been successfully used in the detection of numerous different RNA and DNA viruses, bacteria, fungi, parasites and cytokines. For example, NASBA protocols have been described for human immunodeficiency virus type 1 (Romano et al., 1996), simian immunodeficiency virus (Romano et al., 2000. J. Virol. Methods 86 (1), 61-70), cytomegalovirus (Blok et al., 1999. Transplant. Proc. 31 (1-2), 308-309), hepatitis C virus (Damen et al., 1999. J. Virol. Methods 82 (1), 45-54), Epstein-Barr virus (Hayes et al., 1999. Mol. Pathol. 52 (2), 97-103), measles (Chadwick et al., 1998. J. Virol. Methods 70 (1), 59-70), varicellazoster (Mainka et al., 1998. J. Med. Virol. 56 (1), 91-98), human rhinovirus (Samuelson et al., 1998. J. Virol. Methods 71 (2), 197-209), human papillomavirus type 16 (Smits et al., 1995. J. Virol. Methods 54 (1), 75-81), potato leafroll virus (Leone et al., 1997. J. Virol. Methods 66 (1), 19-27), *Salmonella enterica* (Simpkins et al., 2000. Lett. Appl. Microbiol. 30 (1), 75-79), *Chlamydia trachomatis* (Mahony et al., 2001. J. Clin. Microbiol. 39 (4), 1429-1435), *Campylobacter jejuni* (Uyttendaele et al., 1997. Int. J. Food Microbiol. 37 (1), 13-20), *Mycobacterium leprae* (van der Vliet et al., 1996. Int. J. Lepr. Other Mycobact. Dis. 64 (4), 396-403), *Listeria monocytogenes* (Uyttendaele et al., 1995. Int. J. Food Microbiol. 27 (1), 77-89), *Candida* spp (Borst et al., 2001. Diagn. Microbiol. Infect. Dis. 39 (3), 155-160), *Aspergillus* spp (Loeffler et al., 2001. J. Clin. Microbiol. 39 (4), 1626-1629), *Plasmodium falciparum* (Schoone et al., 2000. J. Clin. Microbiol. 38 (11), 4072-4075), macrophage-derived chemokine mRNA (Romano et al., 2001. Cytokine 13 (6), 325-333), tissue factor mRNA (van Deursen et al., 1999. Nucleic Acids Res. 27 (17), e15) and human TNF-alpha mRNA (Darke et al., 1998. J. Immunol. Methods 212 (1), 19-28), among others. The NASBA technique can be applied to many other situations where nucleic acid amplification is required. The method as commercially available detects the amplified target nucleic acid with an oligonucleotide probe labelled with the electrochemiluminescent (ECL) molecule ruthenium bipyridine (e.g. Romano et al, 1996). However, the ECL technique requires the use of a dedicated detection device that is relatively expensive, not widely available, has relatively low sample throughput and is relatively costly to run. An alternative method of detecting the amplified nucleic acid products from NASBA using cheaper, high throughput, and more readily available detection methods and devices would be beneficial. Such a method is an enzyme-linked probe capture assay, which can be performed in a 96-well microtitre plate format. Standard microtitre plate spectrophotometers are readily available for detection purposes. One of the principle features of NASBA is the incorporation of an oligonucleotide sequence complementary to a chosen oligonucleotide detection probe during the amplification process. This allows the user to detect amplified nucleic acid in a highly specific manner.

Likely targets for NASBA include organisms, such as bacteria, viruses, fungi, and parasites. Influenza A viruses infect a variety of animals, including humans, pigs, horses, sea mammals, and birds. Phylogenetic studies of influenza A viruses have revealed species-specific lineages of viral genes. It has been postulated that aquatic birds are the source of all influenza viruses in other species. The type A influenza virus genome comprises eight single-stranded RNA gene segments that encode ten different proteins (Swayne and Suarez, 2000. Rev. Sci. Tech. 19 (2), 463-482). The proteins can be divided into surface and internal proteins. The surface proteins include haemagglutinin (HA), neuraminidase (NA) and matrix 2 proteins. The HA and NA proteins provide the most important antigenic sites for the production of a protective immune response, primarily in the form of neutralising antibody. There is a great deal of antigenic variation among these proteins, with 15 HA and nine NA subtypes being recognised, based on haemagglutination- inhibition (HI) and neuraminidase-inhibition (NI) tests, respectively.

In 1997 poultry in Hong Kong were infected with highly pathogenic H5N1 avian influenza. The virus contributed to the deaths of six people. As a control measure, all poultry in Hong Kong were eradicated. The virus returned in 2001 causing great economic hardship to poultry farmers and retailers. There exists the need for the routine screening for H5 virus in animals, particularly poultry, in helping to prevent the spread of the virus. Thus a method to accurately detect the presence of H5 in poultry samples would provide material benefits to the poultry industry by identifying infected birds more rapidly than existing antibody-based methods.

The haemagglutinin RNA is translated into a single precursor polypeptide, termed HA0, approximately 556 residues in length (Zambon, 1999. J. Antimicrob. Chemother. 44 (Suppl B), 3-9). A detection method utilising all or part of the conserved region of the influenza haemagglutinin gene would be a useful means of detecting the presence or absence of specific subtypes of influenza A in a biological sample.

Many methods for viral identification are currently used, including cell culture, haemagglutination-inhibition, fluorescent antibody and enzyme immunoassay, and reverse transcriptase polymerase chain reaction (RT-PCR). However, these methods all share the same problems - they have relatively low sensitivity and low specificity. In addition, the detection time may be too long for routine detection purposes, and such methods are relatively difficult to use. Furthermore, as the target of immunodiagnostic assays is usually a specific protein, the underlying genetic nature of a target may not be obtained directly. Finally, the initial derivation of antibodies is ultimately dependent upon the antigenicity of the protein in the immune host animal and therefore, cross-reactivity may occur.

Although virus culture is an accurate and low cost detection method, it is relatively labour intensive and requires a lot of space for incubation. The culturing process may be slow and cannot meet the demand of daily inspection. In addition, virus culture cannot provide the detection results directly and has to reply upon further confirmation by other detection methods, which may be very expensive. Enzyme-linked probe capture assay protocols are generally simple to use and materials and equipment are readily available. Enzyme-linked probe capture assay is a robust technique that has many applications. The principal use of enzyme-linked probe capture assay is to detect protein antigens.

Specific reports relevant to this application include US5783391 (Rossi et al), which describes an NASBA-based detection process. However, US5783391 does not employ an enzyme-linked probe capture assay -based detection step. In addition, numerous reports cover the use of enzyme-linked probe capture assays in detecting countless different biological entities. None of these documents teach the method of the current application nor use the methodology of US5783391 with an enzyme-linked probe capture assay based detection system.

US patent US 5,639,609 describes a method for immobilizing nucleic acids by hybridizing the nucleic acid with a capture probe.

A method for rapidly detecting specific nucleic acid targets in a high throughput format, such as an enzyme-linked probe capture assay, is desirable.

### Object of the invention

One object of the invention is to provide a process for rapidly detecting nucleic acid target molecules, which are the NASBA amplified reaction products of the avian influenza A virus subtype H5 in a high throughput format with high sensitivity.

Another object of the invention is to provide a user-friendly kit for rapidly detecting nucleic acid target molecules, which are the NASBA amplified reaction products of the avian influenza A virus subtype H5 in a high throughput format with high sensitivity.

### Summary of the invention

The present invention provides a process for detecting a nucleic acid target molecule, which comprises the following steps:
(A) capturing the nucleic acid molecule with a capture probe so as to immobilize the nucleic acid target molecule, wherein the capture probe includes:(i) a nucleic acid sequence complementary to at least a portion of the nucleic acid target molecule; and (ii) a label for binding to an immobiliser;
(B) allowing a detection probe to bind to the immobilized nucleic acid target molecule in step (A), wherein the detection probe includes:(i') a nucleic acid sequence complementary to at least a portion of the nucleic acid target molecule; and (ii') a label for binding to a signal generator;
(C) generating and detecting the signal.
wherein the nucleic acid target molecule is the NASBA amplified reaction product of the avian influenza A virus subtype H5 wherein the primers for NASBA include a nucleic acid sequence complementary to a conserved part of the avian influenza virus subtype H5 haemagglutinin gene located within 100 nt either side of the HA1/HA2 cleavage site.
Preferably, steps (A) and (B) are carried out simultaneously.

The present invention also provides a kit for detecting a nucleic acid target molecule, which comprises: (A) a capture probe for capturing the nucleic acid target molecule, which includes: (i) a nucleic acid sequence complementary to at least a portion of the nucleic acid target molecule; and (ii) a label for binding to an immobiliser; (B) a detection probe for detection of the nucleic target molecule, which includes: (i') a nucleic acid sequence complementary to at least a portion of the nucleic acid target molecule; and (ii') a label for binding to a signal generator and (C) primers for NASBA, containing a nucleic acid sequence complementary to at least a portion of a conserved part of the avian influenza virus subtype H5 haemagglutinin gene located within 100 nt either side of the HA1/HA2 cleavage site.

### Brief description of the drawings

Figure 1 shows the flow chart of the overall procedures of the detection of nucleic acid target molecules obtained from a NASBA reaction by the process of this invention.

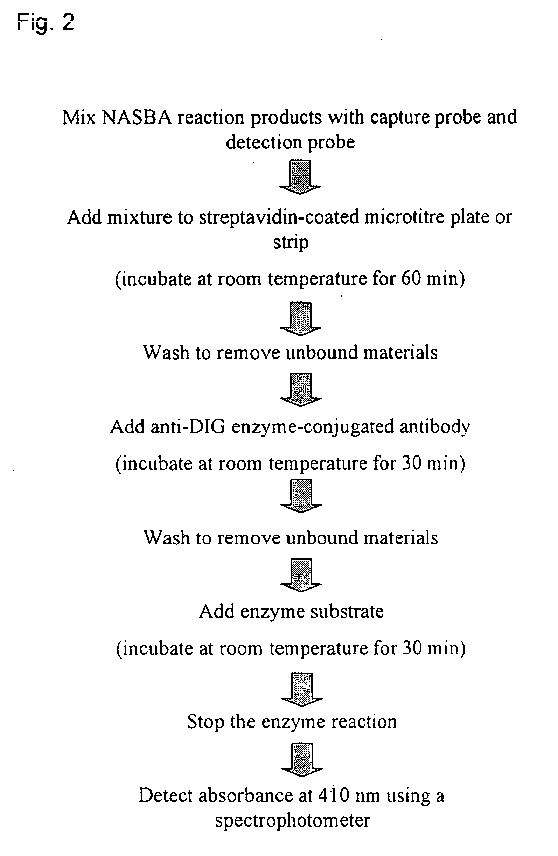

Figure 2 shows the general scheme for the detection of nucleic acid target molecules obtained from a NASBA reaction by the process of this invention.
Figure 3 shows the detailed procedures for the detection of nucleic acid target molecules obtained from a NASBA reaction by the process of this invention.
Figures 4 and 5 show the nucleic acid sequences SEQ ID Nos.1 and 2 , which are used in the DNA molecules of the detection system for detection purposes.

### Detailed description of preferred embodiments

Preferred embodiments of this invention are now described with reference to the figures. List 1 is a part list so that the reference numerals in the figures may be referred to easily.

| **Reference No.** | **Description** |
|---|---|
| 10 | target RNA molecule |
| 12 | capture probe |
| 14 | detection probe |
| 20 | Biotin |
| 22 | Digoxigenin |
| 24 | Microtitre plate |
| 26 | Streptavidin coating |
| 28 | signal generator |

The present invention provides a process for detecting a nucleic acid target molecule, which comprises the following steps:
(A) capturing the nucleic acid molecule with a capture probe so as to immobilize the nucleic acid target molecule, wherein the capture probe includes:
   (i) a nucleic acid sequence complementary to at least a portion of the nucleic acid target molecule; and
   (ii) a label for binding to an immobiliser;
(B) allowing a detection probe to bind to the immobilized nucleic acid target molecule in step (A), wherein the detection probe includes:
   (i') a nucleic acid sequence complementary to at least a portion of the nucleic acid target molecule; and
   (ii') a label for binding to a signal generator;
(C) generating and detecting the signal
wherein the nucleic acid target molecule is the NASBA amplified reaction product of the avian influenza A virus subtype H5 wherein the primers for NASBA include a nucleic acid sequence complementary to a conserved part of the avian influenza virus subtype H5 haemagglutinin gene located within 100 nt either side of the HA1/HA2 cleavage site.

The nucleic acid includes DNA or RNA, provided that the concentration of the nucleic acid target molecule is high enough for detection. For example, the nucleic acid target molecule may be an amplified RNA product through a NASBA method. NASBA is an enzyme-based method for the amplification of nucleic acid. The technique is particularly suitable for the amplification of single stranded RNA and has been successfully used in the detection of numerous different RNA and DNA viruses, bacteria, fungi, parasites and cytokines. The nucleic acid target molecule is the NASBA amplified nucleic acid molecule of the avian influenza virus subtype H5. The primers used in the above-mentioned NASBA reaction are NASBA-PP1 and NASBA-PP2 obtained from Gibco BRL, Life Technologies Inc. (NY, USA).

The capture probe of the invention comprises:
(i) a nucleic acid sequence complementary to at least a portion of the nucleic acid target molecule; and
(ii) a label for binding to an immobiliser;
wherein the nucleic acid sequence complementary to at least a portion of the nucleic acid target molecule is a sequence that is specifically designed for binding to the nucleic acid target molecule. There are no other limitations for the sequence provided that it can bind to the target nucleic acid molecule. The binding generally means that the capture probe can hybridize with the target nucleic acid molecule under conventional hybridization conditions well known in the art. For example, the capture probe hybridizes with the nucleic acid target molecule under a stringent hybridization condition.

The capture probe can be synthesized based on the known sequence of the nucleic acid target molecule by a conventional method such as a chemical synthesis method.

In an embodiment, the capture probe comprises a sequence set forth in SEQ ID No.1, which is used for detecting the avian influenza virus subtype H5. The sequence set forth in SEQ ID No.1 is designed by the inventors and commercially synthesized by the Bioasia Co. (Shanghai, China).

In addition, the capture probe also comprises a label. Preferably, the label is attached to an end of the sequence of the probe. More preferably, the label is covalently attached to the 5'-end of the sequence of the probe. The label of the capture probe can be any one of those that are well known in the art. For example, the capture probe can be labelled with a label selected from a group consisting of biotin, streptavidin, fluorescein, protein A, or other molecule capable of performing the same function. In one embodiment, the label is a biotin label attached to the 5'-end of the sequence of the capture probe.

The immobiliser usually is a solid phase material covered by a ligand that can bind to the label of the capture probe. Preferably, the capture probe is labelled with a biotin and the immobilser is a microtitre plate or strip covered by the ligand specifically against the label, i.e., streptavidin.

Then the reaction container is incubated at room temperature for about 30 min to 2 hours, preferably 1 hour with shaking on a shaking platform. The reaction container is a container that can be easily used on a conventional spectrophotometer, preferably on a common ELISA Reader. For example, the container is a microtube, a 24-well microtitre plate or 96-well microtitre plate. In one embodiment, the container is a 96-well microtitre plate.

Preferably, the reaction container containing the complex of the capture probe and the nucleic acid target molecule is washed with a buffer solution, for example a hybridization buffer, for several times so as to remove the other substances existing in the reaction mixture that may disturb the reaction of the invention, for example the other nucleic acid besides the target nucleic acid, the unbound capture probes, etc.

After immobilization of the nucleic acid target molecule to an immobliser through the action of the capture probe, the reaction mixture is subjected to binding to a detection probe, wherein the detection probe comprises a nucleic acid sequence complementary to at least a portion of the nucleic acid target molecule and a label for binding to a signal generator.

The sequence of the detection probe is a sequence that is specifically designed for binding to at least a portion of the nucleic acid target molecule. The preparation of the sequence can be carried out by a conventional method in the art such as a chemical synthesis method. Preferably, for the detection of NASBA reaction products, a sequence is specifically introduced into the amplified products, so that the amplified nucleic acid comprises a sequence that is complementary to the sequence of the detection probe. The benefit of the above process lies in that the sequence of a commercial probe can be introduced into the amplified products and so that a commercial probe can be used and there is no need for the synthesis of a new probe. In one embodiment of the invention, the sequence of the detection probe comprises a sequence set forth in SEQ ID NO. 2.

The label of the detection probe can be any suitable markers that are well known in the art, such as digoxigenin (DIG), biotin, streptavidin et al. The signal generator may be radioactive (e.g. ³²P), chemiluminescent (e.g.luciferin/luciferase), fluorescent (e.g. fluorescein), enzymatic (e.g.alkaline phosphatase, horeseradish peroxidase, beta-glucuronidase), or eletrochemiluminescent molecule (e.g. ruthenium bipyridine). In one embodiment, the signal generator comprises an anti-DIG enzyme conjugated antibody (suitably diluted in hybridization buffer).

Then the container is incubated at room temperature for about several minutes to several hours, preferably 30 mins with shaking.

Preferably, the unbound signal generator such as the unbound antibody is removed by rinsing the container of reaction multiple times with a buffer such as 1x Tris-buffered saline (1×TBS). Preferably, the rinse is repeated once more.

Preferably, the capture probe and detection probe are added together to the nucleic acid target molecules contained in the container.

Then the substrate solution is added to the reaction mixture. After incubation for an appropriate length of time, preferably 30 min with shaking, the enzyme reaction is stopped by the addition of stop solution. In one embodiment, the signal generator is an anti-DIG alkaline phosphatase. The substrate p-Nitrophenyl phosphate and the stop solution is 3M NaOH.

Then the signal is read on a common spectrophotometer, such as an ELISA Reader.
The present invention also provides a kit for carrying out the above-mentioned process. The kit comprises: (A) a capture probe for capturing the nucleic acid target molecule, which includes: (i) a nucleic acid sequence complementary to at least a portion of the nucleic acid target molecule; and (ii) a label for binding to an immobiliser;(B) a detection probe for detection of the nucleic target molecule, which includes: (i') a nucleic acid sequence complementary to at least a portion of the nucleic acid target molecule; and (ii') a label for binding to a signal generator and (C) primers for NASBA, containing a nucleic acid sequence complementary to at least a portion of a conserved part of the avian influenza virus subtype H5 haemagglutinin gene located within 100 nt either side of the HA1/HA2 cleavage site. Preferably, the kit also comprises other components such as enzyme substrate, and/or stop solution and/or hybridization buffer.

The sequences of the capture probe and detection probe are specifically designed based on the specific nucleic acid target molecule. The enzyme substrate and the stop solution change according to the signal generator used in the reaction. The hybridization buffer is that commonly used in the art, such as 20×SSPE.

The present invention is now illustrated by the following non-limiting examples. It should be noted that various changes and modifications could be applied to the following example and processes without departing from the scope of this invention. Therefore, it should be noted that the following example should be interpreted as illustrative only and not limiting in any sense.

The present invention provides a process for the detection of NASBA amplified reaction products of avian influenza A virus subtype H5 using an enzyme-linked probe capture assay of the present invention.

The concentration of influenza A virus or other organism in a biological sample, for example blood, may be very low such that detection of the presence of the organism's RNA may not be performed on the biological sample directly. In order to increase the number of infectious organism RNA molecules to a sufficient amount for detection purposes, a suitable amplification technology is required. Nucleic acid sequence-based amplification (NASBA) is known to be a flexible technology with particular use for the amplification of RNA. The amplified RNA molecules may then be detected by suitable technology. Enzyme-linked probe capture assay is a rapid, highly sensitive and highly specific method that may be applied to the detection of specific nucleic acid targets following their amplification by NASBA. Results can be obtained in as little as one day.

Avian influenza virus contains its genetic material in the form of eight single strands of ribonucleic acid (RNA). The RNA of all members of avian influenza A contains the genes necessary for their reproduction and one of the essential genes is called haemagglutinin. This gene is approximately 1756 nucleotides in length, with the nucleotides are numbered from the 5' end of the molecule.

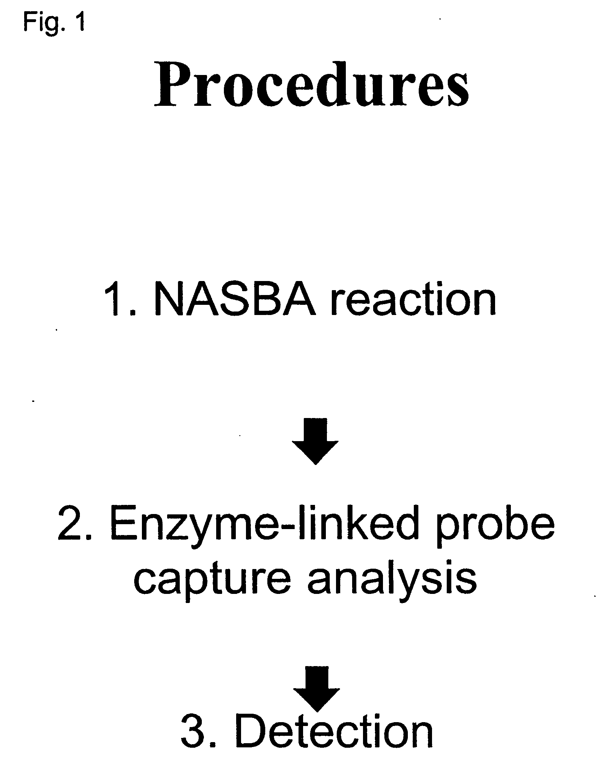

Figure 1 shows the overall procedures for the detection of specific nucleic acid targets by the detection kit. As shown in Figure 1, the target nucleic acid molecules, which are in the form of a single strand of RNA, is firstly extracted from a biological sample. The compatible biological sample types may include blood, serum/plasma, peripheral blood mononuclear cells/peripheral blood lymphocytes (PBMC/PBL), sputum, urine, faeces, throat swabs, dermal lesion swabs, cerebrospinal fluids, cervical smears, pus samples, food matrices, and tissues from various parts of the body including brain, spleen, and liver. Other samples that have not been listed may also be applicable.

After the target RNA molecules are extracted from the biological sample the amount of RNA molecules in the sample may not be sufficient to be detected. Therefore, a portion of the RNA molecule is replicated to a target nucleic acid molecule by NASBA. The target nucleic acid molecules may then be detected by suitable methods, for example enzyme-linked probe capture assay.

After the overall procedures of the detection kit of the invention described, the details of each procedure will now be discussed.

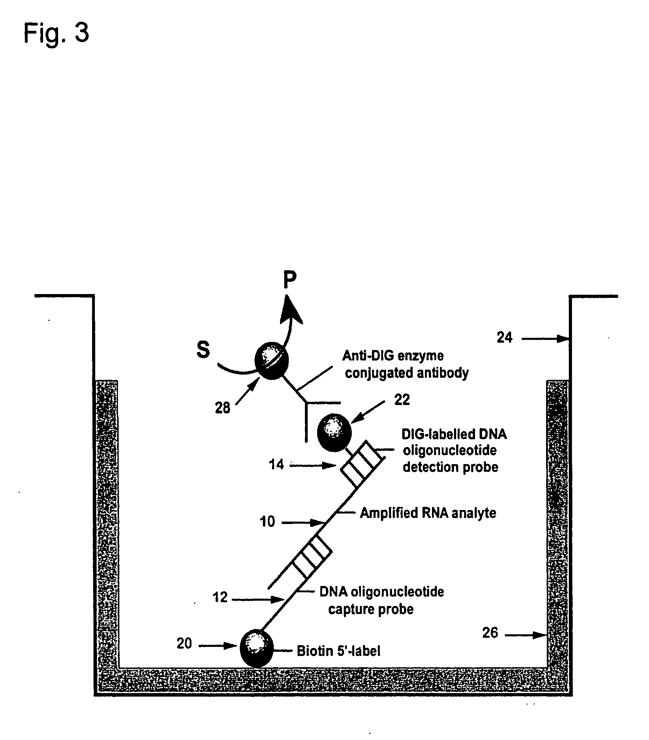

The detection of the target RNA molecule (10) is illustrated in Figure 3. The target RNA molecule (10) is contained in a mixture together with other undesired components including the unamplified nucleic acids contained in the original sample, the primers used to generate the target molecule, the unreacted nucleotides, and most importantly, the unbound detection molecules. Therefore, the target RNA molecule (10) may be immobilised by a capture molecule, for example the capture probe (12), such that the undesired components may be washed away. The capture probe (12) is capable of binding to the target RNA molecule (10). This may be achieved by including a nucleic acid sequence encoding a portion of RNA sequence that is complementary to that encoded in the target RNA molecule (10). The capture probe (12) is further attached to an immobiliser (24), which may immobilise the target RNA molecule (10) so that other undesired components may be washed away. The immobiliser (24) as shown in Figure 3 is a streptavidin-coated microtitre plate or strip or similar surface.

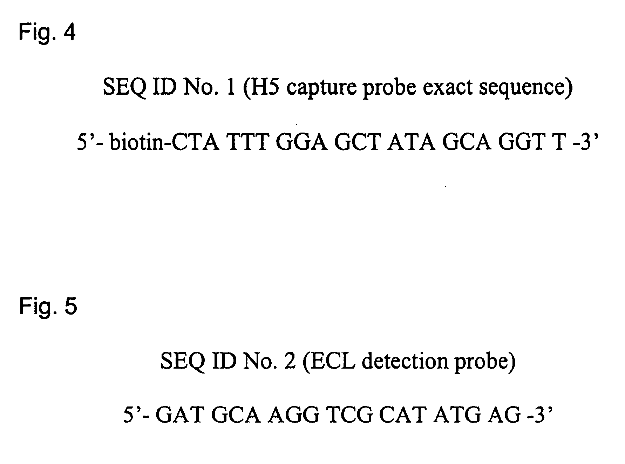

To initiate the nucleic acid detection process a portion of the amplified nucleic acid from a NASBA reaction is mixed with probe solution (containing capture probe (12) and detection probe (14)) and hybridisation buffer in an appropriate container, for example a 1.5 ml microcentrifuge tube. In this embodiment, the capture probe is labelled at its 5'-end with biotin (20). As an alternative it may be possible to replace the biotin with another label capable of performing the same function, i.e. the immobilisation of the target RNA molecule using, for example, labels such as digoxigenin, streptavidin, protein A. In this case the immobilising agent must be changed to allow the binding of the labelled capture probe. The biotin-labelled capture probe (12) comprises the DNA sequence set forth in SEQ ID No. 1 (Figure 4). As an alternative, the capture probe contains a nucleic acid sequence complementary to any portion of the amplified target RNA molecule. The target RNA molecule (10) may be detected by binding to the detection probe (14). In this embodiment, the detection probe is labelled with digoxigenin (DIG) (22). As an alternative it may be possible to replace DIG with other labels, for example biotin, streptavidin, fluorescein, protein A, or other molecule capable of performing the same function, i.e. binding to the signal generator. The sequence of the detection probe (14) may be complementary to any region of the amplified RNA product (10) whose ends are defined by the oligonucleotide primers used to amplify the target molecule. However, the detection probe (14) sequence cannot overlap that of the capture probe (12), as this would affect the interaction of the amplified RNA with the capture probe and vice versa.

As shown in Figure 3, the target RNA molecule (10) may be immobilised together with the detection probe (14). Therefore, there may be no restriction on the timing of the addition of the capture probe (12) into the mixture. The capture probe (12) may be added after or before the addition of the detection probe (10), as long as the capture probe (12) is added before the washing step.

The DIG-labelled detection probe (14) comprises the DNA sequence set forth in SEQ ID No. 2 (Figure 5). As an alternative, any nucleic acid sequence complementary to the target RNA molecule that does not interfere with the binding of the capture probe may serve as a suitable detection probe.

SEQ ID No.1 is subject to change if other capture probe nucleic acid sequences are used. In this embodiment, the immobilisation of the target RNA molecule occurs on a 96-well plastic microtitre plate (24). As an alternative, it may be possible to replace the 96-well microtitre plate with other materials, for example 8-well microtitre strips or other surface capable of performing the same function. Each sample to be tested is added to a separate well of a 96-well streptavidin-coated microtitre plate (24). The microtitre plate (24) with added samples is incubated at room temperature for one hour on a shaking platform. The incubation allows the biotin-labelled capture probe (12) to attach to the streptavidin coating (26) of the microtitre plate (24). In addition, the amplified nucleic acid target molecule (10) from the NASBA reaction hybridises with the biotin-labelled capture probe (12) and the DIG-labelled detection probe (14) during the incubation.

It is important that any unbound molecules (excess amplified nucleic acid target molecule (10), unbound capture probe (12) and unbound detection probe (14) and their complexes) are removed to prevent them interfering with the detection process. Consequently, each well of the microtitre plate (24) is rinsed multiple times with hybridisation buffer.

In this embodiment the signal generator (28) comprises an anti-DIG enzyme-conjugated antibody. As an alternative, the signal generator (28) may be radioactive (e.g. ³²P), chemiluminescent (e.g. luciferin/luciferase), fluorescent (e.g. fluorescein), enzymatic (e.g. alkaline phosphatase, horseradish peroxidase, beta-glucuronidase), or electrochemiluminescent molecule (e.g. ruthenium bipyridine). The detection process requires anti-DIG enzyme-conjugated antibody (suitably diluted in hybridisation buffer) to be incubated with the immobilised nucleic acid complex present in each well of the streptavidin-coated microtitre plate (24). To allow maximal interaction of the antibody with its target antigen the microtitre plate (24) containing the antibody solution is incubated at room temperature for 30 minutes on a shaking platform.

It is important to remove unbound antibodies, as these would interfere with the detection reaction. Consequently, the microtitre plate (24) is rinsed multiple times with 1 x Tris-buffered saline (×TBS).

The amplified nucleic acid target molecule (10) is detected by the addition of substrate solution to each well of the microtitre plate (24). The microtitre plate (24) with added substrate solution is incubated at room temperature for 30 minutes on a shaking platform. After incubation has proceeded for the appropriate length of time, the enzyme reaction is stopped by the addition of stop solution to each well of the microtitre plate (24). The substrate is converted by the conjugated enzyme to yield a product that may be detected in a suitable detection device, for example a microtitre plate spectrophotometer. Thus, under standard conditions, only microtitre plate wells containing RNA target molecules immobilised by capture probe and hybridised to detection probe will produce a signal greater than the background.

As the nucleic acid sequences of the capture probe (12) and detection probe (14) are now known, the synthesis of the corresponding complementary DNA molecules will be apparent to one skilled in the art. Such complementary DNA molecules may be used as templates in the synthesis of the capture probe (12) and detection probe (14).

The present invention is now illustrated by the following non-limiting examples.

### Examples

### Example 1: Detection of the NASBA amplified product of Avian influenza A subtype H5

### Materials and Methods

### a. Viruses

The viruses used in the present invention were isolated by intra-allantoic cavity inoculation of 9-11 day-old chicken embryos (Anon, 1992) at the Castle Peak Verternary Lab (Agriculture, Fisheries and Conservation Department, Kong Kong SAR, China).

### b. Sequence alignment and primer selection

The nucleotide sequences of the haemagglutinin gene from about 50 avian influenza A subtype H5 isolates obtained from GenBank were aligned using the BioEdit software program (Hall, 1997). Conserved sequences within 100nt either side of the HA1/HA2 cleavage site was used for primer selection.

### c. Nucleic acid isolation

Briefly, one volume of clinical specimen (whole blood, plasma, serum, sputum, cells, tissue homogenate, cerebrospinal fluid, etc.) was added to nine volumes of lysis buffer. The sample was mixed gently by vortex mixing. This inactivated infectious virus and stabilized the nucleic acids by denaturing nucleases. Acid-treated silica (50µl, 1mg/ml) was added to the lysate. The sample was kept at room temperature for 10 minutes and vortexed vigorously every two minutes. The liberated influenza virus RNA segments bound to the silica and collected in the solid phase. The silica and nucleic acid complex was pelleted by centrifugation for 30 seconds at 10000 g and washed repeatedly (twice with 5.25 M CuSCN, 50 mM Tris,pH 6.4, 20 mM EDTA; twice with 70% ethanol, and once with acetone). The acetone was evaporated from the silica pelleted by warming the sample in a 56 °C water bath for 10 minutes. DEPC-treated water (50 µl) was added to the dry pellet and incubated in a 56 °C water bath for 10 minutes. The tube was centrifuged for one minute at 10000 g to separate the silica from the water containing the eluted nucleic acid.

### d. NASBA primers

Two pairs of DNA oligonucleotide primers were used in the present invention. The primers used for amplifying the subtype H5 were NASBA-PP1 and NASBA-PP2 obtained from Gibco BRL, Life Technologies Inc. (NY, USA). e. Amplification by NASBA

5 µl of nucleic acid extract, 10 µl of a mixture containing 80 mM Tris, pH 8.3, 24 mM MgCl₂, 140 mM KCl, 10 mM DTT, 2 mM each dNTP, 4 mM each NTP, 30% DMSO, and 0.4 µM each primer was added. This mixture was heated for 65C for five minutes in a water bath, and then cooled to 41 °C for five minutes. Once cool, 5 µl enzyme mix (6.4 units/µl T7 RNA polymerase, 103 units/µl AMV-RT, 0.02 units/µl RNase H, and 0.42 g/µl BSA) was added and the reaction incubated at 41 °C for 90 minutes in a water bath. The final volume was 20 µl.

### f. Preparation of the probe solution

The capture probe comprises a sequence set forth in SEQ ID No. 1 and a biotin label at the 5'-end. The detection probe comprises a sequence set forth in SEQ ID No. 2 and a digoxigenin label at the 5'-end. The sequences of the capture probe and detection probe are designed by the inventors and commercially synthesized by Bioasia Co. (Shanghai, China). Then the capture probe and detection probe are mixed together to obtain a mixed probe solution, wherein the probe solution comprises 0.26 µM biotinylated capture probe and 0. 26 µM DIG-labelled detection probe. The probes used for capturing and detecting the amplified target RNA molecule were obtained from Gibco BRL, Life Technologies Inc. (NY, USA).

### g. Capturing and detection of the NASBA amplified products

The probe solution is added to the NASBA amplified products to capture and detect the amplified nucleic acid, then the above reaction mixture is added to a streptavidin coated microtitre plate and incubated at room temperature for 60 mins. The microtitre plate is washed with hybridization buffer for 2-3 times (20×SSPE), the alkaline phosphatase conjugated anti-DIG antibody is added to the reaction mixture and incubated at room temperature with shaking for 30 mins. Then the microtitre plate is washed 2-3 times to remove unbound materials. The enzyme substrate (p-Nitrophenyl phosphate) is added and the microtitre plate is incubated at room temperature for 30 mins. The reaction is stopped by adding 3M NaOH and the absorbance at 410 nm is detected using a standard microtitre plate spectrophotometer.

The haemagglutinin gene of avian influenza isolates was amplified by the NASBA technique using a pair of DNA oligonucleotide primers according to well-established methods. One primer (NASBA-PP1) contained a 5' extension allowing the incorporation of the T7 bacteriophage RNA polymerase binding site, while the other primer (NASBA-PP2) contained a 5' extension allowing the incorporation of a detection sequence. Following the NASBA reaction the amplification mixture was analysed according to the procedures described above. The amplified H5 target RNA molecules are detected by the enzyme-linked probe capture assay method. Non-H5 material is not detected by the process described. The detection of the NASBA products was as follows:
1. 2 µl probe solution, 5 µl NASBA product and 93 µl hybridization buffer were added to a 1.5 ml microcentrifuge tube;
2. the mixture of 1 was added to a 96-well microtitre plate;
3. the microtitre plate was incubated at room temperature for 1hr with shaking on a shaking platform;
4. the microtitre plate was rinsed with 200 µl hybridization buffer;
5. the microtitre plate was soaked with 200 µl 1×TBS for 5 min;
6. the supernatant was discarded and the microtitre plate was rinsed with 200 µl 1×TBS again;
7. 100 µl diluted anti-DIG alkaline phosphatase (2mg/ml) was added to the microtitre plate;
8. the microtitre plate was incubated at room temperature for 30 min with shaking;
9. the microtitre plate was rinsed once with 200 µl 1×TBS;
10. The microtitre plate was soaked for 5min with 200 µl 1×TBS;
11. step 10 was repeated once more;
12. the contents in the wells of the microtitre plate were discarded and the microtitre plate was rinsed with 200 µl 1 xTBS;
13. 200µl substrate solution (2mg/ml p-Nitrophenyl phosphate) was added to the microtitre plate;
14. the microtitre plate was incubated at room temperature for 30 min with shaking;
15. 100 µl stop solution (3M NaOH) was added to the microtitre to cease the color development;
16. the signals were read on a standard microtitre plate spectrophotometer. 100 µl substrate solution plus 100 µl stop solution were used as background control.
The results are shown in the following table.

| | Signal (OD 410nm) after 30 min | | | | | |
|---|---|---|---|---|---|---|
| | Undiluted H5 template | 1/10 diluted H5 template | 1/100 diluted H5 template | Control: non-H5 amplicon | Control: no added amplicon | Background (Substrate only) |
| 1 | 1.584 | | | | 0.090 | 0.072 |
| 2 | | 1.497 | 0.303 | | 0.096 | 0.066 |
| 3 | | 1.884 | 0.370 | 0.218 | 0.245 | 0.080 |

As shown in the above example, it can be realised that the detection method may be used conveniently in various testing sites including farms. Furthermore, the detection method is relatively easier to use than existing methods, and may be able to provide the detection results in a shorter time - the detection results may be available within one day if desired.

### Example 2. A kit for the detection of the amplified NASBA products of avian influenza subyype H5 virus using an enzyme-linked capture probe assay

The kit comprises the following components contained in separate containers:
1) probe solution, which contains 0.26 µM biotinylated capture probe and 0.26 µM DIG-labelled specific probe dissolved in DEPC water, wherein the capture probe comprised a sequence set forth in SEQ ID NO.1 (Figure 4) and the detection probe comprised a sequence set forth in SEQ ID NO.2 (Figure 5); the sequences in SEQ ID Nos. 1 and 2 were commercially synthesized by Bioasia Co. (Shanghai, China); then the sequences were labelled by biotin and DIG through conventional processes by the manufacture;
2) detection solution, which contained anti-DIG alkaline phosphate (diluted 1:500 in 1 ×Tris buffer saline);wherein the anti-DIG alkaline phosphate was obtained from Sigma Chemical Co. St. Louis, MO, USA;
3) substrate solution, which contained 2 mg/ml p-Nitrophenyl phosphate (pNPP) in Tris buffer; wherein the substrate p-Nitrophenyl phosphate was obtained from Sigma Chemical Co . St. Louis, MO, USA;
4) stop solution, which contained 3M NaOH;
5) hybridisation solution, which contained 20×SSPE(3M NaCl, 0.02 M EDTA, pH 7.4); pH 7.4, 50 mM Tris-HCl; pH 8.8, 1% (w/v) bovine serum albumin).

It will be apparent to one skilled in the art that the detection probe and capture probe may be also useful in the form of RNA molecules. DNA molecules are preferred due to their enhanced stability. It will also be apparent to one skilled in the art that conjugate pairs of binding agents, other than streptavidin and biotin, may be used as the immobilising agent and the capture probe label.

## Claims

1. A process for detecting a nucleic acid target molecule, which comprises the following steps:
(A) capturing the nucleic acid target molecule with a capture probe so as to immobilize the nucleic acid target molecule, wherein the capture probe includes:
(i) a nucleic acid sequence complementary to at least a portion of the nucleic acid target molecule; and
(ii) a label for binding to an immobiliser;
(B) allowing a detection probe to bind to the immobilized nucleic acid target molecule in step (A), wherein the detection probe includes:
(i') a nucleic acid sequence complementary to at least a portion of the nucleic acid target molecule; and
(ii') a label for binding to a signal generator;
(C) generating and detecting the signal
- wherein the nucleic acid target molecule is the NASBA amplified reaction product of the avian influenza A virus subtype H5 wherein the primers for NASBA include a nucleic acid sequence complementary to a conserved part of the avian influenza virus subtype H5 haemagglutinin gene located within 100 nt either side of the HA1/HA2 cleavage site.

2. The process of claim 1, wherein the nucleic acid sequences of the capture probe and detection probe are DNA sequences.

3. The process of claim 1, wherein the signal generator is a radioactive, chemiluminescent, fluorescent, enzymatic, or electrochemiluminescent molecule.

4. The process of claim 1, wherein the immobiliser is a solid phase material that is covered by the ligand of the label of the capture probe.

5. The process of claim 1, wherein the label of the capture probe is selected from the group consisting of streptavidin, biotin, digoxigenin and protein A.

6. The process of claim 1, wherein the label of the capture probe is biotin and the immobiliser is covered by streptavidin.

7. The process of claim 1, wherein the signal generator is an enzyme conjugated antibody that is specific against the label of the detection probe.

8. The process of claim 1, wherein the detection probe is labelled with digoxigenin and the signal generator is an enzyme-conjugated anti-DIG antibody.

9. A process of claim 1, which further comprises a washing step after steps A and/or B so as to remove the unbound materials.

10. The process of claim 1, wherein steps (A) and (B) are carried out simultaneously.

11. The process of claim 1, wherein the capture probe comprises a sequence set forth in SEQ ID No. 1, and the detection probe comprises a sequence set forth in SEQ ID No. 2.

12. The process of claim 11, wherein the capture probe is labelled with biotin.

13. The process of claim 11, wherein the detection probe is labelled with digoxigenin.

14. The process of claim 11, wherein the immobiliser is a microtitre plate covered by streptavidin.

15. A kit for detecting a NASBA amplified nucleic acid target molecule of the avian influenza A virus subtype H5, which comprises:
(A) a capture probe for capturing the nucleic acid target molecule, which includes:
(i) a nucleic acid sequence complementary to at least a portion of the nucleic acid target molecule of the avian influenza A virus subtype H5; and
(ii) a label for binding to an immobiliser;
(B) a detection probe for detecting the nucleic target molecule, which includes:
(i') a nucleic acid sequence complementary to at least a portion of the NASBA amplified nucleic acid target molecule;
(ii') a label for binding to a signal generator, and
(C) primers for NASBA, containing a nucleic acid sequence complementary to at least a portion of a conserved part of the avian influenza virus subtype H5 haemagglutinin gene located within 100 nt either side of the HA1/HA2 cleavage site.

16. The kit of claim 15, further comprising an enzyme substrate solution and/or a buffer solution.

17. The kit of claim 15, wherein the signal generator is a radioactive, chemiluminescent, fluorescent, enzymatic, or electrochemiluminescent molecule.

18. The kit of claim 15, wherein the immobiliser is a solid phase material that is covered by the ligand of the label of the capture probe.

19. The kit of claim 15, wherein the label of the capture probe is selected from a group consisting of streptavidin, biotin, digoxigenin and protein A.

20. The kit of claim 15, wherein the label of the capture is biotin and the immobiliser is covered by streptavidin.

21. The kit of claim 15, wherein the signal generator is an enzyme conjugated antibody that is specific against the label of the detection probe.

22. The kit of claim 15, wherein the detection probe is labelled with digoxigenin and the signal generator is an enzyme conjugated anti-DIG antibody.

23. The kit of claim 15, wherein the capture probe comprises a sequence set forth in SEQ ID NO.1 and the detection probe comprises a sequence set forth in SEQ ID NO.2.

## Patentansprüche

1. Verfahren zum Nachweis eines Nukleinsäure-Zielmoleküls, umfassend die folgenden Schritte:
(A) Einfangen des Nukleinsäure-Zielmoleküls mit einer Fängersonde, um das Nukleinsäure-Zielmolekül zu immobilisieren, wobei die Fängersonde
(i) eine zum Nukleinsäure-Zielmolekül wenigstens teilweise komplementäre Nukleinsäuresequenz und
(ii) eine Markierung für die Bindung an einen Immobilisator enthält;
(B) Gestatten der Bindung einer Nachweissonde an das immobilisierte Nukleinsäure-Zielmolekül in Schritt (A), wobei die Nachweissonde
(i') eine zum Nukleinsäure-Zielmolekül wenigstens teilweise komplementäre Nukleinsäuresequenz und
(ii') eine Markierung für die Bindung an einen Signalerzeuger enthält;
(C) Erzeugen und Nachweisen des Signals,
- wobei es sich bei dem Nukleinsäure-Zielmolekül um das NASBA-amplifizierte Reaktionsprodukt des Subtyps H5 des Vogelgrippe-A-Virus handelt, wobei die Primer für NASBA eine zu einem konservierten Teil des Hämagglutinin-Gens des Subtyps H5 des Vogelgrippe-A-Virus komplementäre Nukleinsäuresequenz enthalten, der innerhalb von jeweils 100 Nt vor und hinter der HA1/HA2-Schnittstelle liegt.

2. Verfahren nach Anspruch 1, wobei es sich bei den Nukleinsäuresequenzen der Fängersonde und der Nachweissonde um DNA-Sequenzen handelt.

3. Verfahren nach Anspruch 1, wobei es sich bei dem Signalerzeuger um ein radioaktives, chemilumineszierendes, fluoreszierendes, enzymatisches oder elektrochemilumineszierendes Molekül handelt.

4. Verfahren nach Anspruch 1, wobei es sich bei dem Immobilisator um ein Festphasenmaterial handelt, das mit dem Liganden der Markierung der Fängersonde bedeckt ist.

5. Verfahren nach Anspruch 1, wobei die Markierung der Fängersonde aus der Gruppe Streptavidin, Biotin, Digoxigenin und Protein A ausgewählt ist.

6. Verfahren nach Anspruch 1, wobei es sich bei es sich bei der Markierung der Fängersonde um Biotin handelt und der Immobilisator mit Streptavidin bedeckt ist.

7. Verfahren nach Anspruch 1, wobei es sich bei dem Signalerzeuger um einen enzymkonjugierten Antikörper handelt, der Spezifität gegen die Markierung der Nachweissonde aufweist.

8. Verfahren nach Anspruch 1, wobei die Nachweissonde mit Digoxigenin markiert ist und es sich bei dem Signalerzeuger um einen enzymkonjugierten Anti-DIG-Antikörper handelt.

9. Verfahren nach Anspruch 1, bei dem man ferner nach Schritt (A) und/oder (B) zur Abtrennung der nicht gebundenen Materialien einen Waschschritt durchführt.

10. Verfahren nach Anspruch 1, wobei die Schritte (A) und (B) gleichzeitig durchgeführt werden.

11. Verfahren nach Anspruch 1, wobei die Fängersonde eine Sequenz gemäß SEQ ID Nr. 1 und die Nachweis-sonde eine Sequenz gemäß SEQ ID Nr. 2 umfaßt.

12. Verfahren nach Anspruch 11, wobei die Fängersonde mit Biotin markiert ist.

13. Verfahren nach Anspruch 11, wobei die Nachweissonde mit Digoxigenin markiert ist.

14. Verfahren nach Anspruch 11, wobei es sich bei dem Immobilisator um eine mit Streptavidin bedeckte Mikrotiterplatte handelt.

15. Kit zum Nachweis eines NASBA-amplifizierten Nukleinsäure-Zielmoleküls des Subtyps H5 des Vogelgrippe-A-Virus, wobei der Kit folgendes umfaßt:
(A) eine Fängersonde zum Einfangen des Nukleinsäure-Zielmoleküls, die
(i) eine zum Nukleinsäure-Zielmolekül des Subtyps H5 des Vogelgrippe-A-Virus wenigstens teilweise komplementäre Nukleinsäuresequenz und
(ii) eine Markierung für die Bindung an einen Immobilisator enthält;
(B) eine Nachweissonde zum Nachweisen des Nukleinsäure-Zielmoleküls, die
(i') eine zum NASBA-amplifizierten Nukleinsäure-Zielmolekül wenigstens teilweise komplementäre Nukleinsäuresequenz und
(ii') eine Markierung für die Bindung an einen Signalerzeuger enthält; und
(C) Primer für NASBA, die eine zu einem konservierten Teil des Hämagglutinin-Gens des Subtyps H5 des Vogelgrippe-A-Virus wenigstens teilweise komplementäre Nukleinsäuresequenz enthalten, die innerhalb von jeweils 100 Nt vor und hinter der HA1/HA2-Schnittstelle liegt.

16. Kit nach Anspruch 15, ferner eine Enzymsubstratlösung und/oder eine Pufferlösung umfassend.

17. Kit nach Anspruch 15, wobei es sich bei dem Signalerzeuger um ein radioaktives, chemilumineszierendes, fluoreszierendes, enzymatisches oder elektrochemilumineszierendes Molekül handelt.

18. Kit nach Anspruch 15, wobei es sich bei dem Immobilisator um ein Festphasenmaterial handelt, das mit dem Liganden der Markierung der Fängersonde bedeckt ist.

19. Kit nach Anspruch 15, wobei die Markierung der Fängersonde aus der Gruppe Streptavidin, Biotin, Digoxigenin und Protein A ausgewählt ist.

20. Kit nach Anspruch 15, wobei es sich bei es sich bei der Markierung der Fängersonde um Biotin handelt und der Immobilisator mit Streptavidin bedeckt ist.

21. Kit nach Anspruch 15, wobei es sich bei dem Signalerzeuger um einen enzymkonjugierten Antikörper handelt, der Spezifität gegen die Markierung der Nachweissonde aufweist.

22. Kit nach Anspruch 15, wobei die Nachweissonde mit Digoxigenin markiert ist und es sich bei dem Signalerzeuger um einen enzymkonjugierten Anti-DIG-Antikörper handelt.

23. Kit nach Anspruch 15, wobei die Fängersonde eine Sequenz gemäß SEQ ID Nr. 1 und die Nachweissonde eine Sequenz gemäß SEQ ID Nr. 2 umfaßt.

## Revendications

1. Procédé pour détecter une molécule d'acide nucléique cible, qui comprend les étapes suivantes :
(A) capturer la molécule d'acide nucléique cible avec une sonde de capture de manière à immobiliser la molécule d'acide nucléique cible, la sonde de capture comprenant :
(i) une séquence d'acide nucléique complémentaire d'au moins une partie de la molécule d'acide nucléique cible ; et
(ii) une étiquette pour la fixation à un support d'immobilisation ;
(B) permettre à une sonde de détection de se fixer à la molécule d'acide nucléique cible immobilisée à l'étape (A), la sonde de détection comprenant :
(i') une séquence d'acide nucléique complémentaire d'au moins une partie de la molécule d'acide nucléique cible ; et
(ii')une étiquette pour la fixation à un générateur de signal ;
(C) générer et détecter le signal,
- la molécule d'acide nucléique cible étant le produit de la réaction d'amplification NASBA du virus A de la grippe aviaire de sous-type H5, les amorces pour l'amplification NASBA comprenant une séquence d'acide nucléique complémentaire d'une partie conservée du gène de l'hémagglutinine du virus de la grippe aviaire de sous-type H5 située à l'intérieur d'une région de 100 nucléotides de chaque côté du site de clivage HA1/HA2.

2. Procédé suivant la revendication 1, dans lequel les séquences d'acide nucléique de la sonde de capture et de la sonde de détection sont des séquences d'ADN.

3. Procédé suivant la revendication 1, dans lequel le générateur de signal est une molécule radioactive, chimioluminescente, fluorescente, enzymatique ou électrochimioluminescente.

4. Procédé suivant la revendication 1, dans lequel le support d'immobilisation est un matériau à phase solide, qui est recouvert par le ligand de l'étiquette de la sonde de capture.

5. Procédé suivant la revendication 1, dans lequel l'étiquette de la sonde de capture est sélectionnée parmi le groupe consistant en la streptavidine, la biotine, la digoxigénine et la protéine A.

6. Procédé suivant la revendication 1, dans lequel l'étiquette de la sonde de capture est la biotine et le support d'immobilisation est recouvert de streptavidine.

7. Procédé suivant la revendication 1, dans lequel le générateur de signal est un anticorps conjugué d'un enzyme qui est dirigé spécifiquement à l'encontre de l'étiquette de la sonde de détection.

8. Procédé suivant la revendication 1, dans lequel la sonde de détection est marquée à la digoxigénine et le générateur de signal est un anticorps anti-DIG conjugué d'un enzyme.

9. Procédé suivant la revendication 1, qui comprend en outre une étape de lavage après les étapes A et/ou B de manière à éliminer les matériaux non fixés.

10. Procédé suivant la revendication 1, dans lequel les étapes (A) et (B) sont effectuées simultanément.

11. Procédé suivant la revendication 1, dans lequel la sonde de capture comprend une séquence prescrite dans SEQ ID NO. 1, et la sonde de détection comprend une séquence prescrite dans SEQ ID NO. 2.

12. Procédé suivant la revendication 11, dans lequel la sonde de capture est marquée à la biotine.

13. Procédé suivant la revendication 11, dans lequel la sonde de détection est marquée à la digoxigénine.

14. Procédé suivant la revendication 11, dans lequel le support d'immobilisation est une microplaque de titration recouverte de streptavidine.

15. Trousse pour détecter une molécule d'acide nucléique cible du virus de la grippe aviaire A sous-type H5 amplifiée par NASBA, qui comprend :
(A) une sonde de capture pour capturer la molécule d'acide nucléique cible, qui comprend:
(i) une séquence d'acide nucléique complémentaire d'au moins une partie de la molécule d'acide nucléique cible du virus de la grippe aviaire A sous-type H5 ; et
(ii) une étiquette pour fixer à un support d'immobilisation;
(B) une sonde de détection pour détecter la molécule d'acide nucléique cible, qui comprend :
(i') une séquence d'acide nucléique complémentaire d'au moins une partie de la molécule d'acide nucléique cible amplifiée par NASBA ;
(ii')une étiquette pour fixer à un générateur de signal, et
(C) des amorces pour l'amplification NASBA, contenant une séquence d'acide nucléique complémentaire d'au moins une partie d'une région conservée du gène de l'hémagglutinine du virus de la grippe aviaire A sous-type H5 située à l'intérieur d'une région de 100 nucléotides de chaque côté du site de clivage HA1/HA2.

16. Trousse suivant la revendication 15, qui comprend de plus une solution de substrat enzymatique et/ou une solution tampon.

17. Trousse suivant la revendication 15, dans laquelle le générateur de signal est une molécule radioactive, chimioluminescente, fluorescente, enzymatique ou électrochimioluminescente.

18. Trousse suivant la revendication 15, dans laquelle le support d'immobilisation est un matériau à phase solide, qui est recouvert par le ligand de l'étiquette de la sonde de capture.

19. Trousse suivant la revendication 15, dans laquelle l'étiquette de la sonde de capture est sélectionnée parmi le groupe consistant en la streptavidine, la biotine, la digoxigénine et la protéine A.

20. Trousse suivant la revendication 15, dans laquelle l'étiquette de la sonde de capture est la biotine et le support d'immobilisation est recouvert de streptavidine.

21. Trousse suivant la revendication 15, dans laquelle le générateur de signal est un anticorps conjugué à un enzyme qui est dirigé spécifiquement à l'encontre de l'étiquette de la sonde de détection.

22. Trousse suivant la revendication 15, dans laquelle la sonde de détection est marquée à la digoxigénine et le générateur de signal est un anticorps anti-DIG conjugué d'un enzyme.

23. Trousse suivant la revendication 15, dans laquelle la sonde de capture comprend une séquence prescrite dans SEQ ID NO. 1, et la sonde de détection comprend une séquence prescrite dans SEQ ID NO. 2.
